# FASCICULE DE BREVET EUROPEEN

(11) **EP 1 753 347 B1**
(45) Date de publication et mention de la délivrance du brevet: **09.01.2008**
(21) Numéro de dépôt: 05773001.2
(22) Date de dépôt: 17.05.2005
(51) Int. Cl.: A61B 17/12, A61B 17/11

(54) **BANDELETTE ANTIFISTULE.**
ANTIFISTELSTREIFEN
ANTI-FISTULA STRIP

(30) Priorité: 25.05.2004 FR 0405617
(43) Date de publication de la demande: 21.02.2007
(73) Titulaire: Nocca, David Jean-Marie, 34000 Montpellier (FR)
(72) Inventeur: Nocca, David Jean-Marie, 34000 Montpellier (FR)
(74) Mandataire: Eidelsberg, Victor Albert
(86) Numéro de dépôt international: PCT/FR2005/001230
(87) Numéro de publication internationale: WO 2006/000665

(56) Documents cités:
- WO-A-02/076305
- WO-A-03/105703
- US-A- 5 147 389
- US-A- 5 741 283

## Description

L'utilisation d'anastomose digestive ou biliaire, qu'elle soit réalisée manuellement ou bien à l'aide de pince mécanique, est très fréquente en chirurgie viscérale (notamment dans le traitement des occlusions intestinales).

La complication la plus fréquente (et possiblement mortelle) est la survenue d'une fistule anastomotique consécutive à un défaut d'étanchéité de l'anastomose. Celle-ci va provoquer une péritonite par passage du contenu intraluminal dans la cavité péritonéale, toute perforation du tube digestif pouvant être à l'origine de cette même complication.

WO 02/076305 décrit une bandelette tel que celle décrite dans le préambule de la revendication 1.

L'invention pallie ce danger par une bandelette souple, inélastique, d'un seul tenant, en une matière résorbable ayant un axe longitudinal, comprenant une fermeture permettant de réunir ses deux tronçons d'extrémités pour former un anneau, l'un des tronçons d'extrémités étant élargi par rapport à l'autre qui est étroit, caractérisée en ce que le tronçon élargi est percé d'au moins une paire d'orifices de suture , les orifices d'une paire étant disposés symétriquement par rapport à l'axe longitudinal de la bandelette.

Par souple, on entend que la bandelette peut être recourbée en un anneau sans se rompre.

Par le choix d'une matière résorbable, l'implant sera colonisé par les fibroblastes et revascularisé. Les tests expérimentaux ont montré une bonne solidité pour une épaisseur semblable au treillis synthétique tricoté. Par les moyens permettant de réunir les deux extrémités de la bande, on peut adapter le diamètre de l'anneau formé en recourbant la bandelette et en maintenant la forme de manière à assurer une bonne étanchéité.

De préférence, les moyens sont constitués par des conformations de la bandelette soi-même et mieux encore uniquement par des conformations de la bandelette soi-même. C'est ainsi que les moyens peuvent être constitués par une fente près de l'une des extrémités et par une languette conjuguée à l'autre extrémité qui peut être enfilée dans la fente lorsque la bandelette est recourbée en un anneau.

De préférence, la bandelette a une partie d'extrémité de plus grande largeur que la fente, de manière à bien assurer l'étanchéité en regard de la fente. Cette partie d'extrémité de plus grande largeur a avantageusement aux quatre coins des orifices de fixation sur le viscère par un fil y passant. La fente a notamment une largeur de 1 à 2 mm correspondant à l'épaisseur de la bandelette et une longueur juste supérieure à la largeur du tronçon principal de la bandelette.

Pour assurer une bonne étanchéité, il vaut mieux que chaque orifice ait une dimension comprise entre 1 et 3 mm et que les orifices d'une paire soient percés à une distance du bord du tronçon élargi comprise entre 2 mm et 5 mm. On assure ainsi à la fois un bon suturage et une absence de déformation de la bandelette. Il vaut mieux que les orifices d'une paire soient à une distance l'un de l'autre supérieure à la largeur du tronçon étroit pour bien maintenir la bandelette et pour prévenir ainsi tout défaut d'étanchéité.

Suivant un mode de réalisation particulièrement apprécié, le tronçon élargi a une forme ovale, ce qui tout en assurant une bonne étanchéité est moins contondant.

De préférence, un repère visuel s'étend transversalement sur le tronçon étroit, ce qui permet de définir un diamètre minimum de la bandelette après positionnement autour du viscère. Le chirurgien est sûr ainsi de ne pas trop serrer l'organe tubulaire du corps.

Suivant un mode de réalisation préféré, deux orifices d'une paire sont alignés avec la direction longitudinale de la fente et il n'y a que ces deux orifices prévus pour une suture. Le serrage de la bandelette est ainsi bien équilibré et l'étanchéité mieux garantie.

Comme produits résorbables utilisables, on peut citer par exemple :
- du collagène d'origine animale ou humaine, notamment porcine,
- de la sous muqueuse intestinale d'origine animale,
- de la sous muqueuse vésicale d'origine animale et
- du péricarde bovin.

Comme types d'anastomoses et taille minimale de celles-ci pour éviter les sténoses, on peut mentionner :
- l'anastomose colo-rectale et iléo-rectale : taille minimale 15 mm de diamètre interne,
- l'anastomose cholédoco-jéjunale : taille minimale : 6 mm de diamètre interne,
- l'anastomose pancréatico-jéjunale : taille minimale : variable suivant la taille du moignon pancréatique,
- l'anastomose oeso-gastrique: taille minimale 12 mm de diamètre interne,
- l'anastomose gastro-jéjunale : taille minimale 12 mm de diamètre interne.

L'invention vise enfin un procédé d'anastomose digestive ou biliaire ou de traitement des perforations du tube digestif dans lequel on enserre un organe tubulaire du corps d'une bandelette suivant l'invention et on suture la bandelette à un viscère du corps par les orifices de suture de la bandelette.

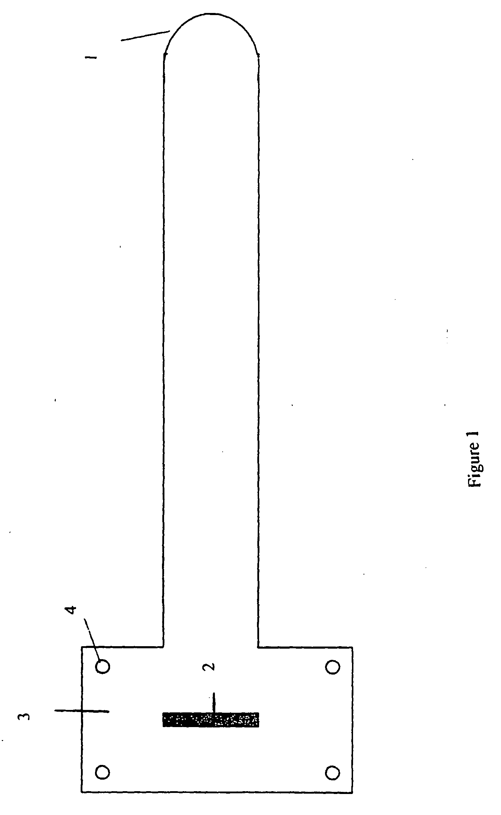

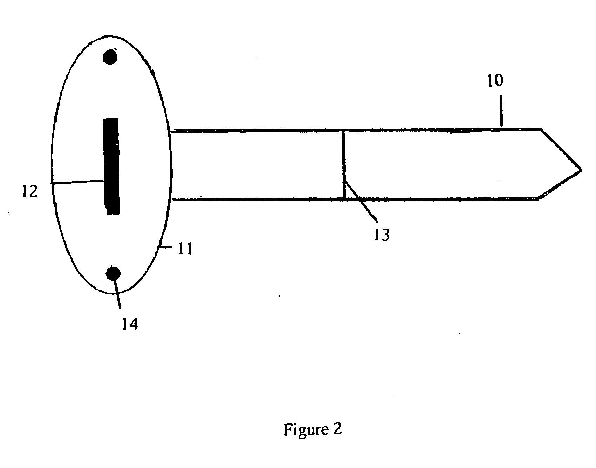

Les figures 1 et 2 du dessin annexé illustrent l'invention.

La figure 1 est une vue en plan d'une bandelette en collagène de porc, de 12 cm de longueur et de 3 cm de largeur. A l'une des extrémités se trouve une languette 1, arrondie de 3 cm de diamètre, tandis qu'à l'autre extrémité est ménagée une fente 2 de 3, 2 cm de longueur s'étendant perpendiculairement à la direction allant d'une extrémité de la bandelette à l'autre telle, qu'après avoir recourbé la bandelette en un anneau, on peut insérer la languette 1 dans la fente 2 en fermant ainsi l'anneau d'une manière étanche et en ayant ainsi la possibilité de régler le diamètre de l'anneau.

La fente 2 est ménagée dans une partie 3 élargie de 3 cm dans le sens de longueur de la bandelette et de 7 cm dans le sens perpendiculaire à la plus grande étendue de la bandelette. Aux quatre coins de la partie 3 sont percés quatre orifices 4 de fixation sur le viscère.

La figure 2 est une vue en plan d'une bandelette préférée suivant l'invention en collagène de porc de 12 cm de longueur indépendamment de la partie pointue de la languette et ayant une partie 10 principale d'une largeur de 1, 5 cm et d'une partie 11 élargie en forme d'ellipse dont le grand diamètre est de 6 cm et le petit diamètre de 3 cm. Dans cette partie 11 élargie est ménagée une fente 12 un peu plus large que le tronçon 10 en sorte que celui-ci peut y passer pour constituer un anneau. A 5,5 cm de la fente 12, est ménagé sur l'anneau, un trait 13 qui permet au praticien de définir un diamètre minimum de l'anneau qu'il ne faut pas dépasser sous peine de trop enserrer l'organe corporel. De part et d'autre de la fente 12 et alignés sur le grand diamètre de l'ellipse, sont ménagés seulement deux orifices 14 de suture de façon symétrique d'une dimension de 2 mm. Les orifices 14 sont circulaires. Le centre du cercle d'un orifice de suture est distant du sommet du grand axe de l'ellipse qui en est le plus proche de 5 mm.

## Revendications

1. Bandelette chirurgicale souple inélastique, d'un seul tenant, en une matière résorbable ayant un axe longitudinal, comprenant une fermeture (2) permettant de réunir ses deux tronçons d'extrémités pour former un anneau, l'un des tronçons d'extrémités (3) étant élargi par rapport à l'autre (1) qui est étroit, **caractérisée en ce que** le tronçon élargi (3) est percé d'au moins une paire d'orifices (4) de suture, les orifices (4) d'une paire étant disposés symétriquement par rapport à l'axe longitudinal de la bandelette.

2. Bandelette suivant la revendication 1, **caractérisée en ce que** chaque orifice (4) a une dimension comprise entre 1 et 3 mm.

3. Bandelette suivant la revendication 1 ou 2, **caractérisée en ce que** le tronçon élargi (11) est de forme ovale.

4. Bandelette suivant l'une des revendications 1 à 3, **caractérisée en ce que** les orifices (4, 14) d'une paire sont percés à une distance du bord du tronçon élargi comprise entre 2 mm et 5 mm.

5. Bandelette suivant l'une des revendications 1 à 4, **caractérisée en ce que** les orifices (4, 14) d'une paire sont à une distance l'un de l'autre supérieure à la largeur du tronçon étroit (1,10).

6. Bandelette suivant l'une des revendications précédentes, **caractérisée par** un repère visuel (13) s'étendant transversalement sur le tronçon étroit (1, 10).

7. Bandelette libre longitudinale suivant la revendication 6, **caractérisée en ce que** le repère visuel (13) est plus près de l'extrémité du tronçon étroit (1, 10) qu'il ne l'est de l'extrémité libre longitudinale du tronçon élargi (3, 11).

8. Bandelette suivant l'une des revendications précédentes, **caractérisée en ce que** la fermeture est constituée par une fente (2, 12) dont la direction longitudinale est perpendiculaire à la direction longitudinale de la bandelette et qui est ménagée dans le tronçon élargi (3, 11) et, par une conformation en languette conjuguée de l'extrémité libre longitudinale du tronçon étroit (1, 10), qui peut être enfilée dans la fente (2, 12) lorsque la bandelette est recourbée en un anneau.

9. Bandelette, suivant la revendication 8, **caractérisée en ce que** deux orifices (4, 14) d'une paire sont alignés avec la direction longitudinale de la fente (2, 12).

10. Bandelette suivant l'une des revendications précédentes, **caractérisée en ce qu'**elle est en collagène.

## Claims

1. Flexible, non-elastic surgical strip, all in one piece, made of resorbable material, having a longitudinal axis, containing a closure (2) allowing one to join its two end pieces into a ring, one of the end pieces (3) being wider than the other (1), which is narrow, **characterised in that** the widened piece (3) is pierced by at least one pair of suture openings (4), the openings (4) of one pair being arranged symmetrically with respect to the longitudinal axis of the strip.

2. Strip according to claim 1, **characterised in that** each opening (4) has a dimension between 1 and 3 mm.

3. Strip according to claim 1 or 2, **characterised in that** the widened piece (11) is of oval shape.

4. Strip according to one of claims 1 to 3, **characterised in that** the opening (4, 14) of one pair are punctured at a distance from the edge of the widened piece between 2 mm and 5 mm.

5. Strip according to one of claims 1 to 3, **characterised in that** the openings (4, 14) of one pair are spaced apart from each other by a distance greater than the width of the narrow piece (1, 10).

6. Strip according to one of the preceding claims, **characterised in that** a visual reference (13) extends transversely on the narrow piece (1, 10).

7. Longitudinal free strip according to claim 6, **characterised in that** the visual reference (13) is closer to the end of the narrow piece (1, 10) than to the longitudinal free end of the widened piece (3, 11).

8. Strip according to one of the preceding claims, **characterised in that** the closure is comprised of a slit (2, 12) whose longitudinal direction is perpendicular to the longitudinal direction of the strip and it is made in the widened piece (3, 11), and by a tongue-like conformation joined to the free longitudinal end of the narrow piece (1, 10), which can be inserted into the slit (2, 12) when the strip is curved into a ring.

9. Strip according to claim 8, **characterised in that** two openings (4, 14) of a pair are aligned with the longitudinal direction of the slit (2, 12).

10. Strip according to one of the preceding claims, **characterised in that** it is made of collagen.

## Patentansprüche

1. Weicher, nicht-elastischer chirurgischer Streifen in einem Stück aus einem resorbierbaren Material mit einer Längsachse, der einen Verschluss (2) aufweist, der es ermöglicht, seine zwei Endabschnitte zur Bildung eines Ringes zu vereinigen, wobei einer der Endabschnitte (3) bezüglich des anderen (1), der schmal ist, verbreitert ist, **dadurch gekennzeichnet, dass** der verbreiterte Abschnitt (3) von mindestens einem Paar von Nähöffnungen (4) durchlöchert ist, wobei die Öffnungen (4) eines Paars symmetrisch in Bezug auf die Längsachse des Streifens angeordnet sind.

2. Streifen gemäß Anspruch 1, **dadurch gekennzeichnet, dass** jede Öffnung (4) eine Abmessung zwischen 1 und 3 mm hat.

3. Streifen gemäß Anspruch 1 oder 2, **dadurch gekennzeichnet, dass** der verbreiterte Abschnitt (11) eine ovale Form hat.

4. Streifen gemäß einem der Ansprüche 1 bis 3, **dadurch gekennzeichnet, dass** die Öffnungen (4, 14) eines Paars in einem Abstand zwischen 2 mm und 5 mm zum Rand des verbreiterten Abschnitts vorgesehen sind.

5. Streifen gemäß einem der Ansprüche 1 bis 4, **dadurch gekennzeichnet, dass** die Öffnungen (4, 14) eines Paars einen Abstand zueinander haben, der größer als die Breite des schmalen Abschnitts (1, 10) ist.

6. Streifen gemäß einem der vorhergehenden Ansprüche, **gekennzeichnet durch** eine visuelle Markierung (13), die sich quer auf dem schmalen Abschnitt (1, 10) erstreckt.

7. Freier Längsstreifen gemäß Anspruch 6, **dadurch gekennzeichnet, dass** die visuelle Markierung (13) dem Ende des schmalen Abschnitts (1, 10) näher ist als dem freien Längsende des verbreiterten Abschnitts (3, 11).

8. Streifen gemäß einem der vorhergehenden Ansprüche, **dadurch gekennzeichnet, dass** der Verschluss von einem Schlitz (2, 12), dessen Längsrichtung senkrecht zur Längsrichtung des Streifens verläuft und der im verbreiterten Abschnitt (3, 11) angeordnet ist, und von einer zugehörigen Laschenanordnung des freien Längsendes des schmalen Abschnitts (1, 10) gebildet wird, der in den Schlitz (2, 12) eingeführt werden kann, wenn der Streifen zu einem Ring gebogen wird.

9. Streifen gemäß Anspruch 8, **dadurch gekennzeichnet, dass** zwei Öffnungen (4, 14) eines Paars mit der Längsrichtung des Schlitzes (2, 12) fluchtend ausgerichtet sind.

10. Streifen gemäß einem der vorhergehenden Ansprüche, **dadurch gekennzeichnet, dass** er aus Collagen besteht.
